# EUROPEAN PATENT APPLICATION

(11) **EP 3 838 305 A1**
(43) Date of publication of application: **23.06.2021**
(21) Application number: 19218014.9
(22) Date of filing: 19.12.2019
(51) Int. Cl.: A61M 1/06, G05B 19/042

(54) **MONITORING FUNCTIONING OF AN OPERATION SOFTWARE PROGRAM OF A BREAST PUMP DEVICE**

(71) Applicant: Koninklijke Philips N.V., 5656 AG Eindhoven (NL)
(72) Inventor: BENTVELSEN, Petrus Henricus Cornelius, 5656 AE Eindhoven (NL); VAN ASSELDONK, Johannes Petrus Antonius Maria, 5656 AE Eindhoven (NL)
(74) Representative: Philips Intellectual Property & Standards

(57) **Abstract**

A breast pump device (1) comprises i) a fluid pressure arrangement (2, 3) configured to interact with a breast from which milk is to be extracted and to realize a pressure cycle at a position where the fluid pressure arrangement (2, 3) is to face the breast, ii) a controller configured to execute an operation software program for controlling operation of the breast pump device (1), the operation software program including instructions to cause the fluid pressure arrangement (2, 3) to realize the pressure cycle, and iii) a monitoring arrangement configured to monitor functioning of the operation software program and to cause release of underpressure in case malfunctioning of the operation software program is detected. As a result, malfunctioning of the operation software program cannot last and any vacuum that may have built up in such a situation is automatically released.

## Description

### FIELD OF THE INVENTION

In the first place, the invention relates to a breast pump device, comprising a fluid pressure arrangement configured to interact with a breast from which milk is to be extracted and to realize a pressure cycle at a position where the fluid pressure arrangement is to face the breast, the pressure cycle involving increase and decrease of a level of underpressure by the fluid pressure arrangement, and a controller configured to execute an operation software program for controlling operation of the breast pump device, the operation software program including instructions to cause the fluid pressure arrangement to realize the pressure cycle.

In the second place, the invention relates to a controller arrangement configured for use in a breast pump device that comprises a fluid pressure arrangement configured to interact with a breast from which milk is to be extracted and to realize a pressure cycle at a position where the fluid pressure arrangement is to face the breast, the pressure cycle involving increase and decrease of a level of underpressure by the fluid pressure arrangement, wherein the controller arrangement includes a controller configured to execute an operation software program for controlling operation of the breast pump device, the operation software program including instructions to cause the fluid pressure arrangement of the breast pump device to realize the pressure cycle.

In the third place, the invention relates to a method of operating a breast pump device that comprises a fluid pressure arrangement configured to interact with a breast from which milk is to be extracted and to realize a pressure cycle at a position where the fluid pressure arrangement is to face the breast, the pressure cycle involving increase and decrease of a level of underpressure by the fluid pressure arrangement, wherein the method comprises a step of executing an operation software program for controlling operation of the breast pump device, the operation software program including instructions to cause the fluid pressure arrangement to realize the pressure cycle.

### BACKGROUND OF THE INVENTION

In general, a breast pump device is a well-known tool for extracting milk from a breast of a lactating woman, or from two breasts simultaneously. Breast pump devices may be used in various situations, for example, if a baby or infant is physically not capable of extracting milk from the breast, or if a mother is separated from her baby or infant and the baby or infant is to be fed with breast milk at a later stage, by the mother or another person. Hence, breast pump devices are used by women to express breast milk at a convenient time, to be stored for later consumption by their/a baby or infant. Breast pump devices may also be helpful in a situation in which it is desired to stimulate and increase milk production in women with a low milk supply or to relieve pressure from engorged breasts.

A breast pump device typically comprises one or two fluid pressure arrangements for interacting with a breast for the purpose of retrieving milk from the breast. The breast pump device may be of an integral design, in which case the device may be suitable to be worn on the body, or the breast pump device may comprise one or two separate units commonly known as expression units or expression kits. Among other things, a fluid pressure arrangement may include a breast-receiving funnel for receiving at least a part of a woman's breast. In any case, the fluid pressure arrangement is configured to realize a pressure cycle at a position where the fluid pressure arrangement is to face the breast, by means of which milk expression from the breast is enabled. The fluid pressure arrangement may be of an electric type and may include a pump/valve arrangement configured to suck air from the air system and to let in air to the air system, for example, wherein the action of sucking air from the air system may be realized on the basis of a pumping action, but manually operated breast pump devices are also known and used in practice. The fact is that by generating a pressure cycle, also referred to as vacuum cycle, possibly accompanied by a certain way of massaging the breast, a simulation of a feeding action is obtained, which triggers the necessary let-down reflex in the lactating woman using the breast pump device.

For the sake of completeness, it is noted that the term "vacuum", or "underpressure", as used in this text refers to a relatively low pressure, i.e. a pressure that is significantly lower than ambient pressure, and that a pressure cycle involves increase and decrease of a level of underpressure by the fluid pressure arrangement. Decrease of a level of underpressure may be decrease to a level of ambient pressure, but this is not necessary. In any case, decrease of a level of underpressure is to be understood so as to be related to an increase of a pressure value, while increase of a level of underpressure is to be understood so as to be related to a decrease of a pressure value, on a scale of pressure values starting from zero.

The invention is in the field of electrically operated breast pump devices. An electrically operated breast pump device is normally equipped with a controller configured to control functioning of the breast pump device by executing one or more software programs, at least an operation software program including instructions to cause the fluid pressure arrangement to realize the pressure cycle. In general, the pressure cycle involves a repetition of a vacuum increasing action and a vacuum reducing action, and transitions between those two actions.

It follows from the foregoing that the controller controls the fluid pressure arrangement by executing the operation software program. If for some reason, malfunctioning (failure) of the operation software program occurs, it may happen that sustained vacuum suction to the breast of a user of the breast pump device is obtained, assuming that the user does not immediately act to remove the device from the breast. For example, software problems may result in continuous operation of the fluid pressure arrangement to increase a level of underpressure and/or failure to decrease the level of underpressure. In such a situation, the user experiences a high level of discomfort and, as a consequence, the breast pump device may be regarded as unsafe. It is therefore an object of the invention to prevent such a situation.

### SUMMARY OF THE INVENTION

The invention provides a breast pump device, comprising i) a fluid pressure arrangement configured to interact with a breast from which milk is to be extracted and to realize a pressure cycle at a position where the fluid pressure arrangement is to face the breast, the pressure cycle involving increase and decrease of a level of underpressure by the fluid pressure arrangement, ii) a controller configured to execute an operation software program for controlling operation of the breast pump device, the operation software program including instructions to cause the fluid pressure arrangement to realize the pressure cycle, and iii) a monitoring arrangement configured to monitor functioning of the operation software program and to cause release of underpressure in case malfunctioning of the operation software program is detected.

On the basis of the fact that according to the invention, a breast pump device is equipped with the monitoring arrangement as defined in the foregoing, it is achieved that any malfunctioning of the operation software program as may occur during a milk expression process performed by means of the breast pump device results in a release of underpressure. Thus, when the invention is put to practice, it is achieved that malfunctioning of the operation software program cannot last and that an action aimed at releasing any underpressure that may have built up in such a situation is taken automatically.

In conformity with the above explanation, it is noted that malfunctioning of the operation software program may be defined as being related to sustained underpressure, which may particularly be sustained underpressure exceeding a predetermined acceptable level. The measures according to the invention provide a way of releasing underpressure in situations in which this is no longer normally done as part of the pressure cycle due to software problems.

In the framework of the invention, it is possible that the monitoring arrangement is configured to initiate a reset procedure of the controller in case malfunctioning of the operation software program is detected, and that the reset procedure of the controller involves an action of causing release of underpressure. Further, the release of underpressure may be part of a standard start-up procedure of the breast pump device, or may be included in a start-up procedure of the breast pump device only if such procedure is initiated after detection of malfunctioning of the operation software program.

In a practical embodiment of the breast pump device according to the invention, the monitoring arrangement comprises a watchdog timer, wherein the operation software program includes an instruction to reset the watchdog timer. If the operation software program does not run properly at some point, the watchdog timer will not be reset in a timely manner. This can be used to advantage as the watchdog timer can be configured to trigger release of underpressure in any suitable way. Hence, when a watchdog timer is applied, detecting malfunctioning of the operational software program is done by detecting expiration of a reset time window of the watchdog timer.

The monitoring arrangement may be included in the controller, i.e. may be internal to the controller, but as an alternative, it is possible that the breast pump device comprises an additional controller (circuit) and that the monitoring arrangement is included in the additional controller (circuit). In the latter case, it is possible to easily add the monitoring arrangement to breast pump devices of an existing design.

It is preferred to have a procedure of causing release of underpressure in case malfunctioning of the operation software program is detected that takes only minimal time. For example, the procedure may be designed such that detecting malfunctioning of the operation software program takes less than 0.5 seconds and that initiating operation of the breast pump device in order to enable release of underpressure takes less than 0.5 seconds as well, so that a total timing of the procedure including the release of underpressure is less than 1 second.

In respect of the structural design of the breast pump device, it is noted that commonly known options are covered by the invention. For example, the fluid pressure arrangement may comprise an air system including an air inlet in communication with the position where the fluid pressure arrangement is to face the breast, and a pump/valve arrangement configured to suck air from the air system and to let in air to the air system. In such a case, the monitoring arrangement may be configured to cause the pump/valve arrangement to let in air to the air system in case malfunctioning of the operation software program is detected, wherein malfunctioning of the operation software program may particularly be related to the pump/valve arrangement continuously sucking air from the air system and/or not letting in air to the air system. In general, the fluid pressure arrangement may include a vacuum pump, a hydraulic pump or any other suitable type of pump of any suitable design. Further, the fluid pressure arrangement may comprise a funnel-shaped breast-receiving shield at the position where the fluid pressure arrangement is to face the breast.

The invention also relates to a controller arrangement configured for use in a breast pump device that comprises a fluid pressure arrangement configured to interact with a breast from which milk is to be extracted and to realize a pressure cycle at a position where the fluid pressure arrangement is to face the breast, the pressure cycle involving increase and decrease of a level of underpressure, wherein the controller arrangement includes a controller configured to execute an operation software program for controlling operation of the breast pump device, the operation software program including instructions to cause the fluid pressure arrangement of the breast pump device to realize the pressure cycle, and a monitoring arrangement configured to monitor functioning of the operation software program and to cause release of underpressure in case malfunctioning of the operation software program is detected.

Options as discussed in the foregoing in respect of the breast pump device are equally applicable to the controller arrangement. These options include i) the option of malfunctioning of the operation software program being related to sustained underpressure, ii) the option of the monitoring arrangement being configured to initiate a reset procedure of the controller in case malfunctioning of the operation software program is detected, and the reset procedure of the controller involving an action of causing release of underpressure, iii) the option of the operation software program including instructions to initiate a start-up procedure of the breast pump device, and the start-up procedure of the breast pump device involving an action of causing release of underpressure, iv) the option of the monitoring arrangement comprising a watchdog timer, and the operation software program including an instruction to reset the watchdog timer, v) the option of the monitoring arrangement being included in the controller or in an additional controller, and vi) the option of causing release of underpressure in case malfunctioning of the operation software program is detected in less than 1 second.

The controller arrangement is suitable for use in any type of breast pump device comprising a fluid pressure arrangement configured to interact with a breast from which milk is to be extracted and to realize a pressure cycle at a position where the fluid pressure arrangement is to face the breast. The controller arrangement may include a communication arrangement for communicating with an external device or system that is separate from the fluid pressure arrangement, and that serves to provide information to a person, including information about detection of software malfunctioning and initiation of a procedure aimed at releasing underpressure. Practical examples of such a device include a smartphone, a smartwatch, a tablet, a laptop and a computer.

The invention further relates to a method of operating a breast pump device that comprises a fluid pressure arrangement configured to interact with a breast from which milk is to be extracted and to realize a pressure cycle at a position where the fluid pressure arrangement is to face the breast, the pressure cycle involving increase and decrease of a level of underpressure by the fluid pressure arrangement, wherein the method comprises the steps of a) executing an operation software program for controlling operation of the breast pump device, the operation software program including instructions to cause the fluid pressure arrangement to realize the pressure cycle, and b) monitoring functioning of the operation software program and causing release of underpressure in case malfunctioning of the operation software program is detected.

As suggested in the foregoing, it is advantageous if a procedure aimed at causing release of underpressure in case malfunctioning of the operation software program is detected is completed in less than 1 second so as to quickly end a situation of malfunctioning software and possible associated sustained vacuum suction, and to quickly restore normal and effective operation of the breast pump device.

A computer program comprising instructions which, when the program is executed by a computer, cause the computer to carry out the method referred to in the foregoing is also covered by the invention.

The above-described and other aspects of the invention will be apparent from and elucidated with reference to the following detailed description of an embodiment of a breast pump device.

### BRIEF DESCRIPTION OF THE DRAWINGS

The invention will now be explained in greater detail with reference to the figures, in which equal or similar parts are indicated by the same reference signs, and in which:
Figure 1 diagrammatically shows a breast pump device comprising an expression unit, a vacuum unit, and a flexible hose interconnecting the expression unit and the vacuum unit, and also diagrammatically shows a smartphone for receiving information from the breast pump device and displaying information to a user; and
Figure 2 diagrammatically shows a number of components of a vacuum unit and a controller arrangement of an embodiment of a breast pump device according to the invention.

### DETAILED DESCRIPTION OF EMBODIMENTS

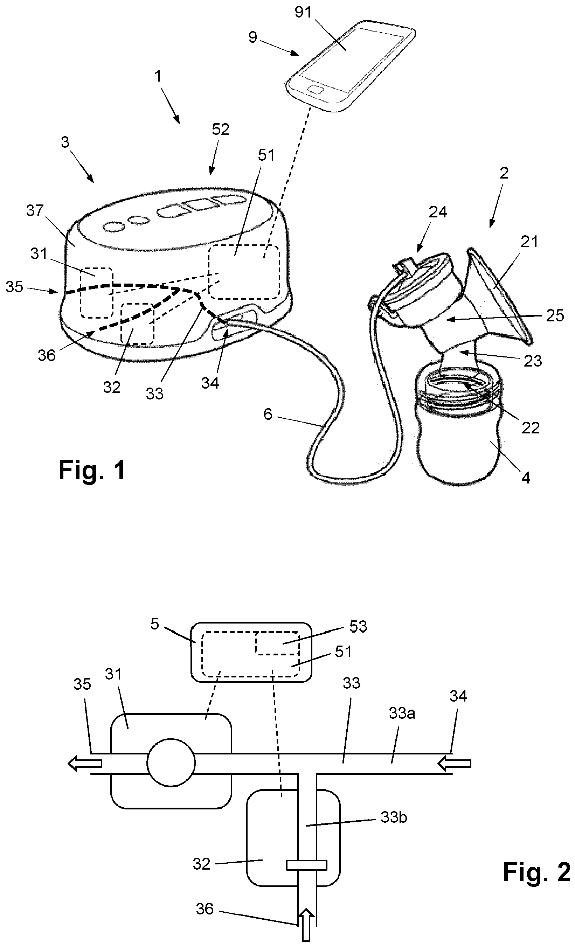

The invention is in the field of breast pump devices. With reference to Figure 1, a general description of an electric breast pump device will be given first so as to provide a clear picture of contextual aspects of the invention.

The breast pump device 1 comprises an expression unit 2 and a vacuum unit 3 for generating a pressure cycle during which a level of vacuum, or underpressure (low pressure with respect to ambient pressure), is alternately increased and decreased. For the purpose of receiving expressed breast milk during operation of the breast pump device 1, a milk receptacle 4 is used which is connectable to the expression unit 2, *e.g.* by screwing, thereby closing a lower end of the expression unit 2. The vacuum unit 3 is an electric vacuum unit and comprises a vacuum pump arrangement 31 and an air valve arrangement 32 for realizing an alternating vacuum during operation, i.e. during milk pumping sessions to be performed by means of the breast pump device 1. The vacuum pump arrangement 31, the air valve arrangement 32 and a controller 51 for realizing proper operation of the vacuum pump arrangement 31 and the air valve arrangement 32 are designed to function in a manner which is well known in the field of breast pump devices, wherein the controller 51 is configured to execute at least an operation software program as will be explained later in more detail.

At the level of the vacuum unit 3, the vacuum pump arrangement 31 acts to realize the pressure cycle in an air system 33 of the vacuum unit 3. The vacuum unit 3 may be electrically connectable to the mains or may include a battery or the like as a source of electric power. The vacuum pump arrangement 31, the air valve arrangement 32 and the controller 51 are diagrammatically depicted in Figure 1 as dashed rectangles. Paths allowing for exchange of control signals etc. between the controller 51 and the vacuum pump arrangement 31 and between the controller 51 and the air valve arrangement 32 are diagrammatically depicted in Figure 1 as a dashed line. Each of the paths may be realized as any suitable type of communication wire, for example. Further, the air system 33 is diagrammatically depicted in Figure 1 as a thick dashed bifurcated line extending from an air inlet 34 of the air system 33 to an air outlet 35 of the air system 33, through the vacuum pump arrangement 31, and also to an air inlet 36 of the air system 33, through the air valve arrangement 32. In order to distinguish the two different air inlets 34, 36 from each other, the first air inlet 34 is referred to as expression unit air inlet 34, and the second air inlet 36 is referred to as ambient air inlet 36.

The expression unit 2 comprises a breast-receiving funnel 21, an aperture acting as a milk outlet 22, and a milk path 23 from the breast-receiving funnel 21 to the milk outlet 22. The breast-receiving funnel 21 is thus in fluid communication with the milk outlet 22 through the milk path 23. The expression unit 2 further comprises an air outlet 24 and an air path 25 from the breast-receiving funnel 21 to the to the air outlet 24.

In Figure 1, the breast pump device 1 is shown in an assembled condition, in which the vacuum unit 3 is connected to the expression unit 2 through a flexible hose 6, wherein the expression unit air inlet 34 of the air system 33 of the vacuum unit 3 is connected to the air outlet 24 of the expression unit 2 so that the pressure cycle that is generated by the vacuum pump arrangement 31 and the air valve arrangement 32 during operation of the breast pump device 1 can be effective in the expression unit 2. The configuration including the flexible hose 6 for interconnecting the expression unit air inlet 34 of the air system 33 of the vacuum unit 3 and the air outlet 24 of the expression unit 2 allows for a remote arrangement of the vacuum unit 3 with respect to the expression unit 2. It is to be noted that the breast pump device 1 can comprise two expression units 2 for enabling a lactating woman using the breast pump device 1 to extract milk from two breasts at the same time, in which case the expression units 2 can share a common vacuum unit 3.

In general, the breast pump device 1 is used to realize milk expression from a woman's breast. To that end, an alternating vacuum (pressure cycle) is applied to the breast. During periods of vacuum, or underpressure, the actual process in which milk is expressed from the breast takes place. Every time that the level of the vacuum is decreased, freshly expressed milk flows to the receptacle 4. At a position between the breast-receiving funnel 21 and the milk outlet 22 of the expression unit 2, a milk valve (not shown) may be present for the purpose of preventing a situation of air leaking in through the milk path 23 during a vacuum suction stage of the pressure cycle. Further, at a position between the breast-receiving funnel 21 and the air outlet 24 of the expression unit 2, a membrane (not shown) may be present for the purpose of preventing a situation of milk reaching the air outlet 24 through the air path 25 during a vacuum decrease stage of the pressure cycle.

Advantageously, as shown, the breast pump device 1 comprises a user interface 52 for allowing a user to control operation of the breast pump device 1. In the shown example, the user interface 52 is arranged on a housing 37 of the vacuum unit 3 and enables a user to provide input to the controller 51. The user interface 52 may be realized in any suitable manner such as through a number of buttons as shown, or through a touch screen, for example. By way of example, it is noted that the user interface 52 may comprise a button for activating a stimulation mode and a number of buttons for choosing one of a number of various expression settings.

Besides the breast pump device 1, Figure 1 shows a smartphone 9 that may be used for providing information during operation of the breast pump device 1 to a user. In particular, the smartphone 9 may be used to run an application designed to display relevant information about the breast pump device 1 and milk pumping sessions performed by means of the breast pump device 1 on the screen 91 of the smartphone 9. Also, the smartphone 9 may be suitable to receive input from a user and transmit the input to the controller 51, in addition to or as an alternative of the user interface 52 on the housing 37 of the vacuum unit 3. A path allowing for exchange of information between the controller 51 and the smartphone 9 is diagrammatically depicted in Figure 1 as a dashed line. The path is preferably realized in a wireless manner, which should not be understood such as to imply that a wired path is not feasible as well.

Figure 2 diagrammatically shows a number of components of a vacuum unit 3 and a controller arrangement 5 of an embodiment of a breast pump device 1 according to the invention. For the sake of clarity, it is noted that it is assumed that the above general description of a breast pump device 1 is entirely applicable to the present embodiment of the breast pump device 1 according to the invention.

In Figure 2, the vacuum pump arrangement 31, the air valve arrangement 32 and the controller arrangement 5 are shown as rectangles, and the paths allowing for exchange of control signals etc. between the controller 51 that is included in the controller arrangement 5 and the vacuum pump arrangement 31 and between the controller 51 and the air valve arrangement 32 are indicated by dashed lines. Further, the air system 33 is depicted as a conduit system having a pump section 33a extending between the expression unit air inlet 34 and the air outlet 35 associated with the vacuum pump arrangement 31 and having a valve section 33b that is open to the pump section 33a and that extends between the pump section 33a and the ambient air inlet 36 associated with the air valve arrangement 32. In Figure 2, a direction in which air sucked from the expression unit 2 is displaced through the pump section 33a of the air system 33 when the vacuum pump arrangement 31 is operated is indicated by means of two horizontal arrows, and a direction of ambient air that is let in to the valve section 33b of the air system 33 when the air valve arrangement 32 is opened is indicated by means of a vertical arrow.

In particular, the controller 51 is configured to execute an operation software program in order to let the breast pump device 1 perform its milk pumping function, the operation software program including instructions to alternately put the vacuum pump arrangement 31 to an operational mode and a non-operational mode of at least reduced air sucking functionality relative to the operational mode, and to alternately put the air valve arrangement 32 to an operational mode and a non-operational mode of at least reduced air inlet functionality relative to the operational mode, respectively. Generally, the operation software program is designed so as to alternately realize the vacuum suction stage at which the vacuum pump arrangement 31 is in the operational mode and the air valve arrangement 32 is in the non-operational mode, and the vacuum decrease stage at which the air valve arrangement 32 is in the operational mode and the vacuum pump arrangement 31 is in the non-operational mode so as to generate a pressure cycle that is suitable for mimicking the suction of a baby or infant at a user's breast.

The controller arrangement 5 further includes a monitoring arrangement 53 that is configured to monitor functioning of the operation software program and to cause release of underpressure in case malfunctioning of the operation software program is detected. On the basis thereof, prolonged faulty operation of the breast pump device 1 and possible associated sustained vacuum suction are avoided. It is practical if the action of causing release of underpressure in case malfunctioning of the operation software program is detected is realized through an action of putting the air valve arrangement 32 to the operational mode. Such action may be performed by initiating a start-up procedure of the breast pump device 1, provided that such start-up procedure includes an instruction to put the air valve arrangement 32 to the operational mode. Alternatively, the start-up procedure does not include an instruction to put the air valve arrangement 32 to the operational mode, so as to exclude unnecessary activation of the air valve arrangement 32, in which case an action of putting the air valve arrangement 32 to the operational mode initiated under the influence of the monitoring arrangement 53 is not performed in the context of a start-up procedure.

The monitoring arrangement 53 may be designed in any suitable way and may comprise a watchdog timer, for example, in which case it is appropriate for the instructions of the operation software program to include an instruction to reset the watchdog timer. It is practical if the controller 51 and/or the monitoring arrangement 53 are accommodated in the housing 37 of the vacuum unit 3, but this is not necessary in the framework of the invention.

The smartphone 9 as shown in Figure 1 or another suitable device can be used to provide information about detection of software malfunctioning and subsequent release of underpressure to a user, besides the information mentioned in the foregoing, wherein it is to be noted that the smartphone 9 or the other suitable device may as well be omitted in the framework of the invention.

It is to be noted that many alternatives to the above-described embodiment of the breast pump device 1 according to the invention are feasible. For example, there is no need to have a separate air valve arrangement 32 in the breast pump device 1. Instead, a pump arrangement having an integrated air valve may be used, or it is even possible to vent air from a pump arrangement at an area that is especially adapted for leaking air to the environment when pumping operation of the pump arrangement is terminated. Also, it is possible for the breast pump device 1 to be of an overall integrated design in which the fluid pressure arrangement 2, 3 does not comprise separate units 2, 3 which are connectable or connected to each other. For example, if the fluid pressure arrangement 2, 3 includes one or more components functioning to realize a pumping action, such components may be integrated in a breast-receiving funnel 21.

The invention is not restricted to a particular operation mode of the breast pump device 1. In this respect, it is noted that the concept of releasing underpressure in case malfunctioning of the operation software program is detected is applicable when the breast pump device 1 functions in an operation mode commonly known as expression mode, and also when the breast pump device 1 functions in an operation mode commonly known as stimulation mode, for example. Both the expression mode and the stimulation mode involve generation of a pressure cycle, wherein the pressure cycle of the expression mode has other characteristics than the pressure cycle of the stimulation mode, especially as far as a relation between level of underpressure and time is concerned.

In case the fluid pressure arrangement 2, 3 comprises two expression units 2, the invention covers both an option of applying the concept of releasing underpressure in case malfunctioning of the operation software program is detected in respect of one of the expression units 2 to only that one of the expression units 2 and an option of applying the concept as mentioned to both expression units 2.

It will be clear to a person skilled in the art that the scope of the invention is not limited to the examples discussed in the foregoing, but that several amendments and modifications thereof are possible without deviating from the scope of the invention as defined in the attached claims. It is intended that the invention be construed as including all such amendments and modifications insofar they come within the scope of the claims or the equivalents thereof. While the invention has been illustrated and described in detail in the figures and the description, such illustration and description are to be considered illustrative or exemplary only, and not restrictive. The invention is not limited to the disclosed embodiments. The drawings are schematic, wherein details which are not required for understanding the invention may have been omitted, and not necessarily to scale.

Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. Any reference signs in the claims should not be construed as limiting the scope of the invention.

Elements and aspects discussed for or in relation with a particular embodiment may be suitably combined with elements and aspects of other embodiments, unless explicitly stated otherwise. Thus, the mere fact that certain measures are recited in mutually different dependent claims does not indicate that a combination of these measures cannot be used to advantage.

The term "comprise" as used in this text will be understood by a person skilled in the art as covering the term "consist of'. Hence, the term "comprise" may in respect of an embodiment mean "consist of', but may in another embodiment mean "contain/include at least the defined species and optionally one or more other species".

The term "fluid" as used in this text will be understood by a person skilled in the art as covering liquids, gases and liquid/gas mixtures. In the context of a breast pump device, a practical example of liquids which may be used in a process of realizing the pressure cycle is milk and a practical example of gases which may be used in a process of realizing the pressure cycle is air.

Notable aspects of the invention can be summarized as follows. A breast pump device 1 comprises i) a fluid pressure arrangement 2, 3 configured to interact with a breast from which milk is to be extracted and to realize a pressure cycle at a position where the fluid pressure arrangement 2, 3 is to face the breast, the pressure cycle involving increase and decrease of a level of underpressure by the fluid pressure arrangement 2, 3, ii) a controller 52 configured to execute an operation software program for controlling operation of the breast pump device 1, the operation software program including instructions to cause the fluid pressure arrangement 2, 3 to realize the pressure cycle, and iii) a monitoring arrangement 53 configured to monitor functioning of the operation software program and to cause release of underpressure in case malfunctioning of the operation software program is detected. As a result, malfunctioning of the operation software program cannot last and any vacuum that may have built up in such a situation is automatically released. Advantageously, the action aimed at releasing vacuum is performed in the same way as normally done during the pressure cycle, so that the action can be performed without a need for an additional valve or the like.

## Claims

1. Breast pump device (1), comprising:
- a fluid pressure arrangement (2, 3) configured to interact with a breast from which milk is to be extracted and to realize a pressure cycle at a position where the fluid pressure arrangement (2, 3) is to face the breast, the pressure cycle involving increase and decrease of a level of underpressure by the fluid pressure arrangement (2, 3),
- a controller (52) configured to execute an operation software program for controlling operation of the breast pump device (1), the operation software program including instructions to cause the fluid pressure arrangement (2, 3) to realize the pressure cycle, and
- a monitoring arrangement (53) configured to monitor functioning of the operation software program and to cause release of underpressure in case malfunctioning of the operation software program is detected.

2. Breast pump device (1) according to claim 1, wherein the monitoring arrangement (53) is configured to initiate a reset procedure of the controller (52) in case malfunctioning of the operation software program is detected, and wherein the reset procedure of the controller (52) involves an action of causing release of underpressure.

3. Breast pump device (1) according to claim 1 or 2, wherein the operation software program includes instructions to initiate a start-up procedure of the breast pump device (1), and wherein the start-up procedure of the breast pump device (1) involves an action of causing release of underpressure.

4. Breast pump device (1) according to any of claims 1-3, wherein malfunctioning of the operation software program is related to sustained underpressure.

5. Breast pump device (1) according to any of claims 1-4, wherein the monitoring arrangement (53) comprises a watchdog timer, and wherein the operation software program includes an instruction to reset the watchdog timer.

6. Breast pump device (1) according to any of claims 1-5, wherein the monitoring arrangement (53) is included in the controller (52).

7. Breast pump device (1) according to any of claims 1-5, comprising an additional controller, wherein the monitoring arrangement (53) is included in the additional controller.

8. Breast pump device (1) according to any of claims 1-7, wherein causing release of underpressure in case malfunctioning of the operation software program is detected takes less than 1 second.

9. Breast pump device (1) according to any of claims 1-8, wherein the fluid pressure arrangement (2, 3) comprises an air system (33) including an air inlet (34) in communication with the position where the fluid pressure arrangement (2, 3) is to face the breast, and a pump/valve arrangement (31, 32) configured to suck air from the air system (33) and to let in air to the air system (33), and wherein the monitoring arrangement (53) is configured to cause the pump/valve arrangement (31, 32) to let in air to the air system (33) in case malfunctioning of the operation software program is detected.

10. Breast pump device (1) according to claim 9, wherein malfunctioning of the operation software program is related to the pump/valve arrangement (31, 32) continuously sucking air from the air system (33) and/or not letting in air to the air system (33).

11. Breast pump device (1) according to any of claims 1-10, wherein the fluid pressure arrangement (2, 3) comprises a funnel-shaped breast-receiving shield (21) at the position where the fluid pressure arrangement (2, 3) is to face the breast.

12. Controller arrangement (5) configured for use in a breast pump device (1) that comprises a fluid pressure arrangement (2, 3) configured to interact with a breast from which milk is to be extracted and to realize a pressure cycle at a position where the fluid pressure arrangement (2, 3) is to face the breast, the pressure cycle involving increase and decrease of a level of underpressure by the fluid pressure arrangement (2, 3),
wherein the controller arrangement (5) includes
- a controller (52) configured to execute an operation software program for controlling operation of the breast pump device (1), the operation software program including instructions to cause the fluid pressure arrangement (2, 3) of the breast pump device (1) to realize the pressure cycle, and
- a monitoring arrangement (53) configured to monitor functioning of the operation software program and to cause release of underpressure in case malfunctioning of the operation software program is detected.

13. Method of operating a breast pump device (1) that comprises a fluid pressure arrangement (2, 3) configured to interact with a breast from which milk is to be extracted and to realize a pressure cycle at a position where the fluid pressure arrangement (2, 3) is to face the breast, the pressure cycle involving increase and decrease of a level of underpressure by the fluid pressure arrangement (2, 3),
wherein the method comprises the steps of
a) executing an operation software program for controlling operation of the breast pump device (1), the operation software program including instructions to cause the fluid pressure arrangement (2, 3) to realize the pressure cycle, and
b) monitoring functioning of the operation software program and causing release of underpressure in case malfunctioning of the operation software program is detected.

14. Method of operating a breast pump device (1) according to claim 13, wherein causing release of underpressure in case malfunctioning of the operation software program is detected takes less than 1 second.

15. Computer program comprising instructions which, when the program is executed by a computer, cause the computer to carry out the method of claim 13.
